# EUROPEAN PATENT APPLICATION

(11) **EP 4 079 154 A1**
(43) Date of publication of application: **26.10.2022**
(21) Application number: 21169953.3
(22) Date of filing: 22.04.2021
(51) Int. Cl.: A01N 1/00

(54) **USE OF APOPTOSIS INHIBITORS FOR COLD STORAGE OF BLOOD PLATELETS**

(71) Applicant: DRK-Blutspendedienst Baden-Württemberg-Hessen gGmbH, 60528 Frankfurt am Main (DE); Zentrum für Klinische Transfusionsmedizin Tübingen gGmbH (ZKT), 72076 Tübingen (DE)
(72) Inventor: Bakchoul, Tamam, 72072 Tübingen (DE); Seifried, Erhard, 60320 Frankfurt (DE)
(74) Representative: Krauss, Jan

(57) **Abstract**

The present invention relates to a pharmaceutical composition for cold storing blood platelets, comprising platelets in a suitable storage medium, and at least one inhibitor of platelet apoptosis. Preferably, said apoptosis inhibitor is a selective inhibitor targeting cold-storage induced apoptosis.

## Description

The present invention relates to a pharmaceutical composition for cold storing blood platelets, comprising platelets in a suitable storage medium, and at least one inhibitor of platelet apoptosis. Preferably, said apoptosis inhibitor is a selective inhibitor targeting cold-storage induced apoptosis.

### Background of the invention

Platelet transfusion has become essential therapy in modern medicine. Although the clinical advantage of platelet transfusion has been well established, adverse reactions upon transfusion, especially transmission of bacterial infection, still represent a major challenge. Transfusion of platelet concentrates (PCs) is also essential to reduce blood loss after traumatic injury or to maintain a safe platelet (PLT) count during chemotherapy.

Currently, PCs are stored at room temperature (20-24°C) with constant agitation to ensure adequate PLT recovery, survival and sufficient therapeutic efficacy.

However, storage at room temperature (RT) not only compromises functionality both *in vivo* and *in vitro* ('PLT storage lesions') but also increases the potential risk of microbial growth in case of contamination [1-6]. For these reasons, the shelf life of PLTs is limited, depending on country specific guidelines, to 4-5 days [2]. However, despite limited storage time, the incidence of bacterial contamination of PCs remains high, ranging from 1 to 10 per 50.000 units, which is a major drawback of PLTs stored at RT for clinical use.

Cold storage of PCs at 4°C could be an option to reduce the risk of bacterial growth [7,8]. Recent studies reported that cold-stored PLTs are functionally and metabolically superior to those stored at RT [9-11].

Currently the FDA allows for cold storage of platelets at 1 to 6°C without agitation for up to 3 days for trauma patients (21 CFR 640.24 and 640.25). The Mayo Clinic has implemented use of such 3 day platelets for trauma patients in recent years. The military, after significant research and data collection, submitted a variance request to the FDA. This variance was just approved by the FDA in August 2019 with the statement "To store apheresis platelets at 1-6 °C for up to 14 days without agitation. The cold stored platelet products will be used to treat actively bleeding patient when conventional platelet products are not available, or their use is not practical."

A potential limitation of cold storage is, however, the poorer recovery and survival of PLTs after transfusion. However, this issue is controversial. In fact, some studies showed a decrease in survival of cold-stored PLTs in comparison to RT-stored PLTs [12-14], while other investigators reported that PLTs stored at 4°C can survive in the circulation for several days [15,16]. Refrigerated platelets were reported to be cleared rapidly from circulation, precluding cold storage of platelets for transfusion. Based on these studies, the concept of cold storage had been abandoned in clinical routine.

Recently, cold storage of PCs seasoned a renaissance when PLT storage in additive solutions (PAS) became available. It was demonstrated that apheresis platelet concentrates (APCs) stored at 4°C were detectable in the mouse blood circulation for up to 5 hours implying that platelet degradation was not enhanced. Compared with human platelets stored at RT, platelets stored at 4°C have higher expression of apoptosis marker compared to platelets stored at room temperature, leading to accelerated clearance from the circulation in a humanized animal model (Marini I, et al. Cold storage of platelets in additive solution: the impact of residual plasma in apheresis platelet concentrates. Haematologica. 2019 Jan; 104(1):207-214. doi: 10.3324/haematol.2018.195057. Epub 2018 Aug 16. PMID: 30115655; PMCID: PMC6312032).

Leytin (in: Apoptosis in the anucleate platelet. Blood Rev. 2012 Mar;26(2):51-63. doi: 10.1016/j.blre.2011.10.002. Epub 2011 Nov 4. PMID: 22055392) discloses that, for many years, programmed cell death, known as apoptosis, was attributed exclusively to nucleated cells. Currently, however, apoptosis is also well-documented in anucleate platelets, and extrinsic and intrinsic pathways of apoptosis in nucleated cells and in platelets, platelet apoptosis induced by multiple chemical stimuli and shear stresses, markers of platelet apoptosis, mitochodrial control of platelet apoptosis, and apoptosis mediated by platelet surface receptors PAR-1, GPIIbIIIa and GPIbα. In addition, data is presented on platelet apoptosis provoked by aging of platelets in vitro during platelet storage, platelet apoptosis in pathological settings in humans and animal models, and inhibition of platelet apoptosis by cyclosporin A, intravenous immunoglobulin and GPIIbIIIa antagonist drugs.

Xiang et al. (in: Xiang B, et al. Calcium Ion Chelation Preserves Platelet Function During Cold Storage. Arterioscler Thromb Vasc Biol. 2021 Jan;41(1):234-249. doi: 10.1161/ATVBAHA. 120.314879. Epub 2020 Nov 12. PMID: 33176450) disclose that a cell impermeable calcium ion chelator such as EGTA inhibited platelet activation and apoptosis in vitro, and prolonged life span of cold-stored platelets after transfusion. EGTA-treated, cold-stored platelets, efficiently prevented bleeding. They conclude that the addition of EGTA to platelet products is potentially a simple and effective method to enable cold storage of platelets for transfusion.

Pleines I, et al. (in: Intrinsic apoptosis circumvents the functional decline of circulating platelets but does not cause the storage lesion. Blood. 2018 Jul 12;132(2):197-209. doi: 10.1182/blood-2017-11-816355. Epub 2018 May 21. PMID: 29784641) disclose that BAK/BAX depletion in murine platelets reveals that intrinsic apoptosis is not required for the development of the platelet storage lesion. Apoptotic culling of old platelets from the bloodstream is described as essential to maintain a functional, hemostatically reactive platelet population. Restriction of platelet life span by intrinsic apoptosis is disclosed as pivotal to maintain a functional, hemostatically reactive platelet population, and therefore inhibiting intrinsic apoptosis in blood banked platelets is described as unlikely to yield significant benefit.

As mentioned above, platelet transfusion has become essential therapy in modern medicine. The above art does not provide a conclusive scenario with respect to apoptosis during cold storage of platelets. Stable and safe platelet preparations are still in high demand. It is therefore an object of the present invention, to provide new strategies for storing platelet preparations. Other objects and advantages of the present invention will become apparent to the person of skill when studying the following more detailed description of the present invention, including the Figures and examples.

In a first aspect of the present invention, the above object is solved by a pharmaceutical composition for cold storing blood platelets, comprising platelets in a suitable storage medium, and at least one inhibitor of platelet apoptosis. Preferably, said apoptosis inhibitor is a selective inhibitor targeting cold-storage induced apoptosis, wherein preferably said apoptosis inhibitor is selected from the group of specific RhoA subfamily Rho GTPases inhibitors, RhoA inhibitor G04, specific phosphokinase A (PKA) activators, forskolin, specific caspase-9 inhibitors, z-LEHD-fmk, and combinations thereof, in particular RhoA inhibitor G04 combined with forskolin.

In a second aspect of the present invention, the above object is solved by a method for preparing a pharmaceutical composition according to the first aspect of the invention, comprising providing a sample of blood platelets to be cold-stored in a suitable storage medium, and adding to said blood platelets at least one inhibitor of platelet apoptosis.

In a third aspect of the present invention, the above method according to the second aspect of the invention further comprises the step of cold-storing said pharmaceutical composition, wherein said cold storing is at a temperature of between about 0°C to about 10°C, preferably between about 2°C to about 5°C, and more preferably at about 4°C. The cold-storing of the pharmaceutical composition preferably is for more than about 4 days, preferably for more than about 7 days, more preferably for more than about 10 days, and most preferably for more than about 14 days.

In a fourth aspect of the present invention, the above object is solved by the pharmaceutical composition according to the present invention for use in the prevention and/or treatment of diseases, preferably for use in platelet transfusion.

In a fifth aspect of the present invention, the above object is solved by a method of treating or preventing a disease or condition requiring platelet transfusion, comprising providing to a subject in need thereof an effective amount of the pharmaceutical composition according to the present invention.

In the context of experiments as performed, the inventors investigated the impact of different apoptosis inhibitors on platelet viability and function. The present invention is based on the surprising finding that platelets cold-stored, for example, at 4°C in the presence of apoptosis inhibitors have similar survival and functional properties compared to those stored at room temperature. Thus, inhibition of cold storage induced or related apoptosis was identified as an approach to prevent impaired platelet survival and function after cold storage.

Therefore, the inventors provide an improved pharmaceutical composition for cold-storing blood platelets, comprising platelets in a suitable storage medium, and at least one inhibitor of platelet apoptosis.

In the context of the present invention, cold-storing is generally at temperatures of below room temperature (20°C or 22°C), generally a temperature of between room temperature and freezing, such as at about 0°C to about 10°C, preferably between about 2°C to about 5°C, and more preferably at about 4°C. Cold storage at about 4°C in a regular fridge is most preferred.

The platelets to be stored can be provided in any suitable storage medium for cold storage. A preferred pharmaceutical composition according to the present invention comprises the platelets as a blood platelet concentrate, such as an autologous or apharesis platelet concentrate (APC), platelets in additive solutions (PAS) or a platelet-rich plasma (PRP). Preferably said PRP does not comprise more than about 35% of plasma. Autologous Platelet Concentrate (APC) APC or platelet-rich plasma (PRP) is defined as a substrate that contains a platelet concentration above basal levels, i.e. above the level found in mammalian, such as human peripheral blood.

Platelet additive solutions (PASs) are becoming increasingly popular for storage of platelets, and PAS is steadily replacing plasma as the storage medium of platelets. PASs are electrolyte solutions intended for storage of platelets, and they are used to modulate the quality of the platelets by adding specific ingredients. All currently available PASs contain acetate. Acetate reduces the amount of glucose that is oxidised into lactic acid and thereby prevents the lowering of pH, which decreases platelet quality. Furthermore, the oxidation of acetate leads to the production of bicarbonate, which serves as buffer. The presence of potassium and magnesium in PAS prevents the lowering of pH and reduces the degree of spontaneous activation of the platelets during storage. In the hospital, platelets stored in PAS result in about half of the number of allergic transfusion reactions as compared with platelets in plasma. Recovery and survival after transfusion, as well as corrected count increments, are at least as good for platelets in PAS as for plasma, and recent data suggest they may even be better. Therefore, PAS (e.g. SSP+) or plasma for the storage of platelet concentrates is preferred (van der Meer PF. PAS or plasma for storage of platelets? A concise review. Transfus Med. 2016 Oct;26(5):339-342. doi: 10.1111/tme.12325. Epub 2016 Jun 7. PMID: 27273164).

Preferably, the apoptosis inhibitor to be added is a selective inhibitor targeting cold-storage induced apoptosis in platelets. A variety of references describe factors involved in cold-storage induced apoptosis in platelets (see also above), such as 14-3-3ζ (Yan Y, Xie R, Ning Z. 14-3-3ζ Regulates the Platelet Apoptosis During Storage. Indian J Hematol Blood Transfus. 2020 Apr;36(2):324-329. doi: 10.1007/s12288-019-01229-z. Epub 2019 Nov 14. PMID: 32425384; PMCID: PMC7229142).

Preferably, the apoptosis inhibitor as used in the context of the present invention is selected from the group of specific RhoA subfamily Rho GTPases inhibitors, specific phosphokinase A (PKA) activators, specific caspase-9 inhibitors, specific 14-3-3ζ activators, and combinations thereof (Shang X, et al. Small-molecule inhibitors targeting G-protein-coupled Rho guanine nucleotide exchange factors. Proc Natl Acad Sci U S A. 2013 Feb 19; 110(8):3155-60. doi: 10.1073/pnas.1212324110. Epub 2013 Feb 4. PMID: 23382194; PMCID: PMC3581902; Evelyn CR, et al. CCG-1423: a small-molecule inhibitor of RhoA transcriptional signaling. Mol Cancer Ther. 2007 Aug;6(8):2249-60. doi: 10.1158/1535-7163.MCT-06-0782. PMID: 17699722). Further preferred examples are RhoA inhibitor G04, forskolin, and z-LEHD-fmk. Particularly preferred are combinations, e.g. RhoA inhibitor G04 combined with forskolin, RhoA inhibitor G04 combined with z-LEHD-fmk.

The amounts and concentrations of apoptosis inhibitors to be used can be readily determined by the person of skill, and usually are found at between 0.1 and 500 µM. In case of a synergistic effect as expected for example with RhoA inhibitor G04 combined with forskolin, or RhoA inhibitor G04 combined with z-LEHD-fmk, the individual concentration may be adjusted accordingly.

The blood platelets to be used, stored and/or transfused in the context of the present invention can be autologous or allogenic, and preferred is the pharmaceutical composition according to the present invention, wherein said blood platelets are from a mammalian donor, in particular a rodent or human donor.

The cold-stored pharmaceutical composition according to the present invention exhibits several advantages, for example the cold stored blood platelets in the pharmaceutical composition according to the present invention exhibit a % platelet (PLT) viability on day 10 of more than about 50%, preferably more than about 55%, more preferably more than about 60%.

Further preferably, the cold stored blood platelets in the pharmaceutical composition according to the present invention exhibit a % apoptosis on day 10 of less than about 40%, and preferably less than about 35%. Even further preferably, the cold stored blood platelets in the pharmaceutical composition according to the present invention a % PLT aggregation on day 7 of more than about 60%, and preferably more than about 65%. Respective methods to test these parameters are disclosed herein, and in the respective literature.

Platelet transfusion became an indispensable therapy in the modern medicine. Although the clinical advantage of platelet transfusion is well established, adverse reactions upon transfusion, especially transmission of bacterial infection, still represent a major challenge. While the bacterial contamination is favored by the storage at room temperature, cold storage may represent a solution for this clinically relevant issue. Recently, the inventors showed that a residual plasma content of approximately 35% is feasible for cold storage of PLTs in apheresis-derived platelet concentrates (APCs), with a potential of prolonging shelf life of GMP-produced APCs stored at 4°C for clinical use.

However, since platelets stored at 4°C displayed higher expression of apoptosis markers compared to room temperature and were cleared faster in a humanized mouse model, this approach is still not applicable in clinical setting. In the present invention, the inventors aimed to elucidate whether inhibition of apoptosis might result in better PLT storage and preserve their functions. The inventors tested impact of three different apoptosis inhibitors: an RhoA inhibitor (G04), PKA inhibitor (Forskolin) and Caspase-9 inhibitor (z-LEHD-fmk) to see if loss in PLT viability and function can be restored.

The inventors' results show that RhoA inhibitor can significantly improve PLT lifespan and restore functions even at longer storage durations of 7-10 days. On the other hand, while forskolin could partially improve the PLT viability and reduce apoptosis, no impact was observed on PLT functions. Similarly, no significant positive effects were observed after treatment with Caspase-9 inhibitor z-LEHD-fmk.

Taken together, the data shows that inhibition of apoptosis using RhoA inhibitor G04 in apheresis-derived platelet concentrates in particular in combination with forskolin, is a suitable substitute strategy to store platelets at 4°C for longer durations.

Another aspect of the present invention then relates to a method for preparing a pharmaceutical composition according to the present invention, comprising providing a sample of blood platelets to be cold-stored in a suitable storage medium, and adding to said blood platelets at least one inhibitor of platelet apoptosis.

One preferred approach to prepare platelets is as apheresis-derived platelet concentrates that are collected with FENWAL AMICUS (Amicus, Fresenius Kabi, Bad Homburg, Germany) and stored in plasma or in PAS (SSP+, Macopharma, Langen, Germany), e.g. at 35% final plasma concentration at 4°C and RT.

For preparation, citrate is the preferred anticoagulant for blood collection, as EDTA damages platelets and heparin modifies their function. Citrate allows the rapid generation of platelet-rich plasma (PRP), with a high yield of platelets; however, this method has certain disadvantages. To overcome these problems, a centrifugation technique has been developed for the isolation and washing of platelets from human or rodent blood anticoagulated with acid-citrate-dextrose (ACD). The cells are resuspended in a physiological buffer under well-defined conditions, notably the presence of plasmatic ionic calcium concentrations (2 mM) and the absence of coagulation factors or other plasma components.

Preferred is the method according to the present invention, wherein said blood platelets are from a mammalian donor, in particular a rodent, a humanized rodent, or human donor.

Similar to the above-mentioned, preferred is the method according to the present invention comprising providing the platelets as a blood platelet concentrate, such as an autologous or apharesis platelet concentrate (APC), platelets in additive solutions (PAS) or a platelet-rich plasma (PRP). Preferably said PRP does not comprise more than about 35% of plasma. Autologous Platelet Concentrate (APC) APC or platelet-rich plasma (PRP) is defined as a substrate that contains a platelet concentration above basal levels, i.e. above the level found in mammalian, such as human peripheral blood.

Preferably, the apoptosis inhibitor to be added is a selective inhibitor targeting cold-storage induced apoptosis in platelets. A variety of references describe factors involved in cold-storage induced apoptosis in platelets (see also above), such as 14-3-3ζ (Yan Y, Xie R, Ning Z. 14-3-3ζ Regulates the Platelet Apoptosis During Storage. Indian J Hematol Blood Transfus. 2020 Apr;36(2):324-329. doi: 10.1007/s12288-019-01229-z. Epub 2019 Nov 14. PMID: 32425384; PMCID: PMC7229142).

Preferably, the apoptosis inhibitor as used in the method of the present invention is selected from the group of specific RhoA subfamily Rho GTPases inhibitors, specific phosphokinase A (PKA) activators, specific caspase-9 inhibitors, specific 14-3-3ζ activators, and combinations thereof (Shang X, et al. Small-molecule inhibitors targeting G-protein-coupled Rho guanine nucleotide exchange factors. Proc Natl Acad Sci U S A. 2013 Feb 19;110(8):3155-60. doi: 10.1073/pnas.1212324110. Epub 2013 Feb 4. PMID: 23382194; PMCID: PMC3581902; Evelyn CR, et al. CCG-1423: a small-molecule inhibitor of RhoA transcriptional signaling. Mol Cancer Ther. 2007 Aug;6(8):2249-60. doi: 10.1158/1535-7163.MCT-06-0782. PMID: 17699722). Further preferred examples are RhoA inhibitor G04, forskolin, and z-LEHD-fmk. Particularly preferred are combinations, e.g. RhoA inhibitor G04 combined with forskolin, RhoA inhibitor G04 combined with z-LEHD-fmk.

Another aspect of the present invention then relates to a method of cold-storing a pharmaceutical composition comprising blood platelets, comprising the method according to the present invention, and further comprising the step of cold-storing said pharmaceutical composition, wherein said cold storing is at a temperature of between about 0°C to about 10°C, preferably between about 2°C to about 5°C, and more preferably at about 4°C. Storing in a normal fridge for about 7-10 days is preferred.

Preferred is the method according to the present invention, wherein said cold-storing of the pharmaceutical composition is for more than about 4 days, preferably for more than about 7 days, more preferably for more than about 10 days, and most preferably for more than about 14 days. It was found in the context of the present invention that platelets stored at 4°C in the presence of apoptosis inhibitors have similar survival and functional properties compared to those stored at room temperature.

In the context of the present invention, the term "about" shall mean +/- 10% of the given value, unless indicated otherwise.

Another aspect of the present invention then relates to the pharmaceutical composition according to the present invention as disclosed herein for use in the prevention and/or treatment of diseases, preferably for use in platelet transfusion, more preferably for the treatment of sepsis, cancer, AIDS, leukemia, aplastic anemia, hypersplenism, bone marrow transplant, organ transplant or surgeries like cardiopulmonary bypass, and/or radiation therapy. The attending physician is able to adjust the respective therapy to the pharmaceutical composition according to the present invention. The use may further include the step of testing for the desired PLT functions, as disclosed above.

Another aspect of the present invention then relates a method of treating or preventing a disease or condition related to, accompanied with, and/or caused by a lack of blood platelets, comprising providing to a subject in need thereof an efficient amount of the cold-stored pharmaceutical composition according to the present invention as disclosed herein. The treatment or prevention may be in case of sepsis, cancer, AIDS, leukemia, aplastic anemia, hypersplenism, bone marrow transplant, organ transplant or surgeries like cardiopulmonary bypass, and/or radiation therapy in said subject, e.g. a human patient. The attending physician is able to adjust the respective therapy to the pharmaceutical composition according to the present invention. The use may further include the step of testing for the desired PLT functions, as disclosed above.

In the studies underlying the present invention, the inventors investigated the impact of storage temperature and subsequent apoptosis inhibition on PLT survival and function during cold storage of APCs.

Previously, the inventors showed that APC storage at 4°C enhances PLT functionality in comparison to storage at room temperature, hence significantly improving their clinical advantage. However, cold storage of PLTs lead to poor survival with increased storage duration. Interestingly, significantly higher amounts of apoptotic cells were observed, with decreasing viability and therefore, the inventors speculated if inhibition of apoptosis might improve PLT viability also at longer storage durations. The inventors tested three different inhibitors of apoptosis pathway and analyzed PLT viability as well as function. The results demonstrate that in particular G04, a RhoA inhibitor, significantly restores PLT viability and function.

Attempts to store APCs at 4°C were impeded by an impaired survival of cold-stored PLTs. Consistently, in the context of the invention, PLTs from APCs stored at 4°C always showed inferior survival curves compared to those stored at RT, confirming previous reports on accelerated clearance of cold-stored PLTs [13,14]. Data from animal model suggested that desialylation of GPIb is responsible for the accelerated elimination of cold-stored PLTs [13,24]. However, the inventors found similar beta-galactose exposure on RT- and cold-stored PLTs, which fits the results of clinical studies that showed that reconstitution of sialylation of human PLTs does not prevent the accelerated clearance of cold-stored APCs [25].

Interestingly, the inventors found that cold storage triggers phosphatidylserine exposure indicating higher PLT apoptosis. Although the differences were not always significant, the trend was obvious. This may indicate that impaired *in vivo* survival of cold-stored PLTs is rather caused by apoptosis-related mechanisms. In fact, cold storage has been previously found to induce GPIb alpha-clustering which makes the receptors an initiation site for PLT apoptosis and macrophage recognition [26]. The inhibition of prostacyclin has been suggested to reduce cold-induced changes in GPIb [27], which could be emphasized by the results. Of note, cold-induced apoptosis was not accompanied by higher spontaneous expression of P-selectin or CD63 indicating that apoptosis and activation during PLT storage are mediated by two independent mechanisms. Therefore, selective targeting of cold-induced apoptosis may provide a new approach to reduce PLT storage lesions. In fact, it was recently reported that cold storage of PLTs enhances the vWF binding to GPIb alpha inducing increased intracellular calcium concentration and phosphatidylserine exposure leading to a fast clearance of PLTs finally. The authors demonstrated that the inhibition of this binding and the consequent downstream cascade, using a specific peptide that recognises GPIb alpha, significantly increased recovery and life span of cold-stored PLTs [28]. Another study showed that a specific caspase-3 inhibitor significantly improves PLT functionality and viability during 7 days of storage [29]. Together with the present results, inhibition of apoptosis seems to be a promising approach to reduce cold-stored lesions.

Finally, the inventors investigated whether their research findings could be transferred into routine production and explored the possibility to extent PC shelf life. To exclude influences of different donors, the inventors designed a follow up study where each donor donated double APCs in PAS containing 35% plasma. Each APC was stored either at RT or at 4°C for up to ten days. The inventors found that PLTs from APCs collected and stored under routine blood bank conditions at 4°C maintained functionality better in terms of activation (granule release in response to TRAP) and aggregation compared to those stored at RT. These results correspond to those from the split design study. Taken together, a residual plasma content of ~35% is feasible to accomplish cold storage of PLTs in APCs for clinical use. Our results indicate the potential to prolong shelf life of GMP-produced APCs stored at 4°C for clinical use by inhibiting PLT apoptosis.

In summary, the present invention provides information into the *in vitro* haemostatic function of cold-stored PLTs and suggests that inhibition of apoptosis in PLTs stored at 4°C may become an alternative method to prolong the PLT shelf life and subsequently improve the current standard of care.

The present invention in particular relates to the following items.
Item 1: A pharmaceutical composition for cold storing blood platelets, comprising platelets in a suitable storage medium, and at least one inhibitor of platelet apoptosis.
Item 2. The pharmaceutical composition according to item 1, wherein said cold storing is at a temperature of between about 0°C to about 10°C, preferably between about 2°C to about 5°C, and more preferably at about 4°C.
Item 3. The pharmaceutical composition according to item 1 or 2, wherein said blood platelets are from a mammalian donor, in particular a human donor.
Item 4. The pharmaceutical composition according to any one of items 1 to 3, comprising platelets as a blood platelet concentrate, such as an autologous or apharesis platelet concentrate (APC), platelets in additive solutions (PAS) or a platelet-rich plasma (PRP), wherein preferably said PRP does not comprise more than about 35% of plasma.
Item 5. The pharmaceutical composition according to any one of items 1 to 4, wherein said apoptosis inhibitor is a selective inhibitor targeting cold-storage induced apoptosis, wherein preferably said apoptosis inhibitor is selected from the group of specific RhoA subfamily Rho GTPases inhibitors, RhoA inhibitor G04, specific phosphokinase A (PKA) activators, forskolin, specific caspase-9 inhibitors, z-LEHD-fmk, and combinations thereof, in particular RhoA inhibitor G04 combined with forskolin.
Item 6. The pharmaceutical composition according to any one of items 1 to 5, wherein said cold stored blood platelets show a % platelet (PLT) viability on day 10 of more than about 50%, preferably more than about 55%, more preferably more than about 60%.
Item 7. The pharmaceutical composition according to any one of items 1 to 6, wherein said cold stored blood platelets show a % apoptosis on day 10 of less than about 40%, and preferably less than about 35%.
Item 8. The pharmaceutical composition according to any one of items 1 to 7, wherein said cold stored blood platelets show a % PLT aggregation on day 7 of more than about 60%, and preferably more than about 65%.
Item 9. A method for preparing a pharmaceutical composition according to any one of items 1 to 8, comprising providing a sample of blood platelets to be cold-stored in a suitable storage medium, and adding to said blood platelets at least one inhibitor of platelet apoptosis.
Item 10. The method according to item 9, wherein said blood platelets are from a mammalian donor, in particular a human donor.
Item 11. The method according to item 9 or 10, wherein said blood platelets are a blood platelet concentrate, such as an autologous or apharesis platelet concentrate (APC), platelets in additive solutions (PAS) or a platelet-rich plasma (PRP), or derived therefrom, wherein preferably said PRP does not comprise more than about 35% of plasma.
Item 12. The method according to any one of items 9 to 11, wherein said apoptosis inhibitor is a selective inhibitor targeting cold-storage induced apoptosis, wherein preferably said apoptosis inhibitor is selected from the group of specific RhoA subfamily Rho GTPases inhibitors, RhoA inhibitor G04, specific phosphokinase A (PKA) activators, forskolin, specific caspase-9 inhibitors, z-LEHD-fmk, and combinations thereof, in particular RhoA inhibitor G04 combined with forskolin.
Item 13. The method according to any one of items 9 to 12, further comprising the step of cold-storing said pharmaceutical composition, wherein said cold storing is at a temperature of between about 0°C to about 10°C, preferably between about 2°C to about 5°C, and more preferably at about 4°C.
Item 14. The method according to item 13, wherein said cold-storing of the pharmaceutical composition is for more than about 4 days, preferably for more than about 7 days, more preferably for more than about 10 days, and most preferably for more than about 14 days.
Item 15. The pharmaceutical composition according to any one of items 1 to 8 for use in the prevention and/or treatment of diseases, preferably for use in platelet transfusion.
Item 16. A method of treating or preventing a disease or condition related to, accompanied with, and/or caused by a lack of blood platelets, comprising providing to a subject in need thereof an efficient amount of the cold-stored pharmaceutical composition according to the present invention as disclosed herein.
Item 17. The method according to item 16, wherein said diseases or condition is selected from sepsis, cancer, AIDS, leukemia, aplastic anemia, hypersplenism, bone marrow transplant, organ transplant or surgeries like cardiopulmonary bypass, and/or radiation therapy in said subject, e.g. a human patient.

The present invention will now be described further in the following examples with reference to the accompanying Figures, nevertheless, without being limited thereto. For the purposes of the present invention, all references as cited herein are incorporated by reference in their entireties.
Figure 1 shows that RhoA inhibitor G04 results in increased viability and decreased apoptosis at longer storage durations of APCs.
Figure 2 shows that cold storage in the presence of Rho inhibitor G04 restores loss of δ granule secretion and PLT aggregation.
Figure 3 shows that forskolin treatment partially restores PLT survival and reduces apoptosis in cold stored APCs.

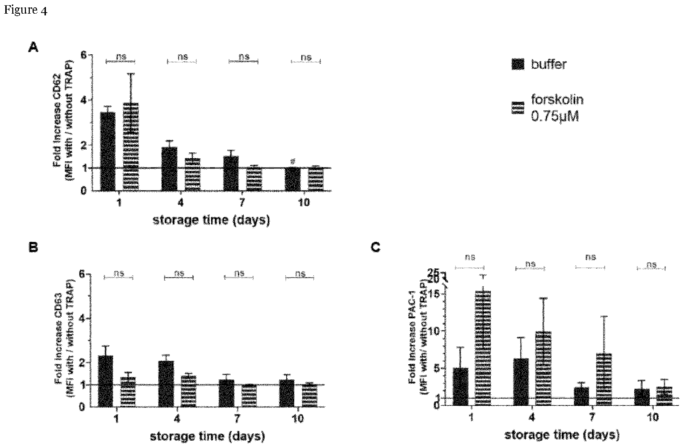

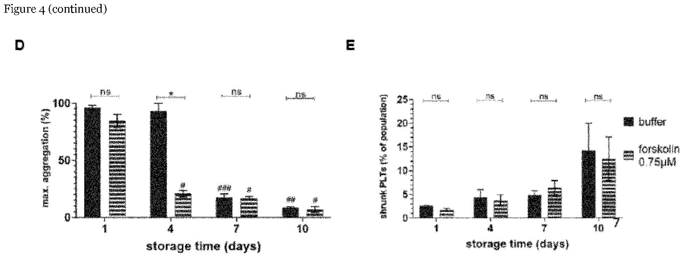

Figure 4 shows that the PKA inhibitor forskolin does not affect deteriorated PLT functions during longer duration of cold storage.
Figure 5 shows that caspase-9 inhibitor z-LEHD-fmk partially restores PLT viability and decreases apoptosis in cold stored APCs.
Figure 6 shows that caspase-9 inhibitor z-LEHD-fmk does not affect the diminished PLT function at longer duration.

### Examples

### Materials and Methods

### Preparation of apheresis platelet concentrates (APCs)

APCs were collected from healthy volunteers according to the German guidelines for haemotherapy. Ten individuals donated two units of APCs collected with FENWAL AMICUS (Amicus, Fresenius Kabi, Bad Homburg, Germany) and stored in plasma or in PAS (SSP+, Macopharma, Langen, Germany) at 35% final plasma concentration at 4°C and RT.

### Treatment of APCs with apoptosis inhibitors

Appropriate concentration for the inhibitors was determined prior to experiments, by titrating different concentrations. Directly after the collection, the APCs were stored for an hour without agitation. APCs were split further into different culture bags under aseptic conditions and stored in sterile, oxygen permeable bag (Fresenius Kabi AG, Bad Homburg, Germany). APCs were treated with 150 µM RhoA inhibitor G04, 0.75 µM Phosphokinase A (PKA) activator forskolin or 40 µM Caspase-9 inhibitor z-LEHD-fmk. All inhibitors were diluted in DMSO, and hence DMSO (600 µM) was used as a vehicle control to exclude any solvent-mediated effects. APCs without any additions were used as a negative control. After 2 hours of treatment at RT, all the samples except the control at RT, were stored at 4 °C under constant gentle agitation. All samples were activated with thrombin receptor-activating peptide TRAP (20µM, HART Biologicals) prior to measurements.

### Determination of apoptosis

PLTs from APCs were washed, resuspended with 1 mM CaCl₂ and stained with Annexin V-FITC (Beckman Coulter) for 60 min at RT. Freshly isolated PLTs were incubated with 10 µM ionomycin (Abcam, Cambridge, UK) and used as positive control.

### Platelet activation and granule release

PLTs (10 x 10⁶/mL) were incubated with TRAP6 (20 µM) for 30 min at 37°C, fixed with 4% PFA for 20 min at RT. The expression of CD62P (CLBThromb/6, Beckman Coulter) and CD63 (CLBGran/12, Beckman Coulter) was determined by FC. The conformational changes of glycoproteins (GPs) IIb/IIIa complex upon activation with TRAP were detected by PAC-1-antibody (BD Bioscience) using FC.

### Platelet aggregation

PLT function was analyzed by FC and light transmission PLT aggregation assay (LTA) using a 4-channel-aggregometer (Labitec, Ahrensburg, Germany). Collagen (8 µg/ml), ristocetin (1.5 µM) (both Mölab, Langenfeld, Germany) or ADP (80 µM, Nycomed, Konstanz, Germany) were used as inductors.

### Testing of cold stored APCs in mice

The cold stored APCs treated with 150µM RhoA inhibitor G04 are tested using a mouse model to analyze their survival and function *in vivo.* Samples pretreated with the inhibitor are collected on day 7 and 10, and transfused into NOD/SCID mice. The recovery rate of PLTs and elimination kinetics is compared with untreated control. Furthermore, the degradation mechanisms of PLT are examined.

### Statistical analysis

Statistical analyses were performed using GraphPad Prism 7 (La Jolla, USA). T-test was used to analyze normally distributes results. Nonparametric tests were used when data failed to follow a normal distribution as assessed by D'Agostino and Pearson omnibus normality test. Group comparison was performed using the Wilcoxon rank-sum test and the Fisher exact test with categorical variables. A p-value <0.05 was assumed to represent statistical significance.

### Cold storage in combination with RhoA inhibitor G04 leads to enhanced viability and decreased apoptosis at longer storage durations

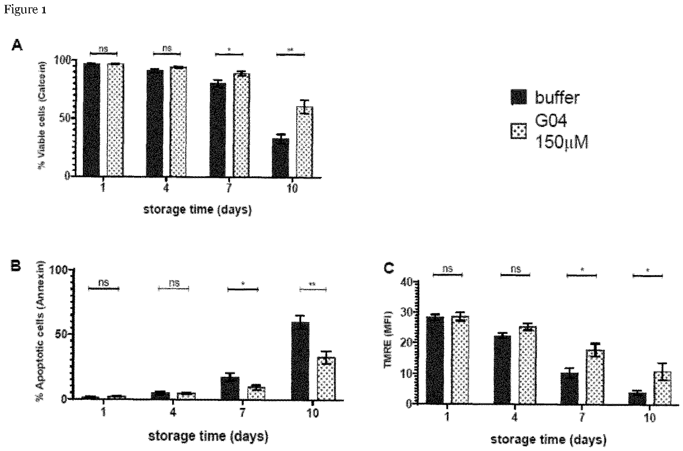

The inventors studied the impact of RhoA inhibitor G04 on platelet viability and apoptosis in cold stored APCs. APCs were incubated with or without 150µM G04 at 4°C under constant gentle agitation. A significant increase in viability was observed (Figure 1A) in G04 treated PLTs at day 7 (% viable cells 89.13 vs 80.42, p=0.040) which further improved at day 10 (60.82 vs 33.07, p=0.0023). In corroboration with this trend, G04 treatment also significantly diminished PLT apoptosis at day 4 (% apoptotic cells 9.3 vs 17.6, p=0.0400) and even more strongly at day 10 (% apoptotic cells 33 vs 60.1, p=0.0020) (Figure 1B). Similar trends were observed in the measurement of mitochondrial transmembrane potential at day 7 and day 10 (ΔΨm MFI 17.88 vs 10.34, p=0.0131 and 11.02 vs 3.95, p=0.034 respectively) (Figure 1C).

### Cold storage in combination with Rho inhibitor G04 restores loss of δ granule secretion and improves PLT function

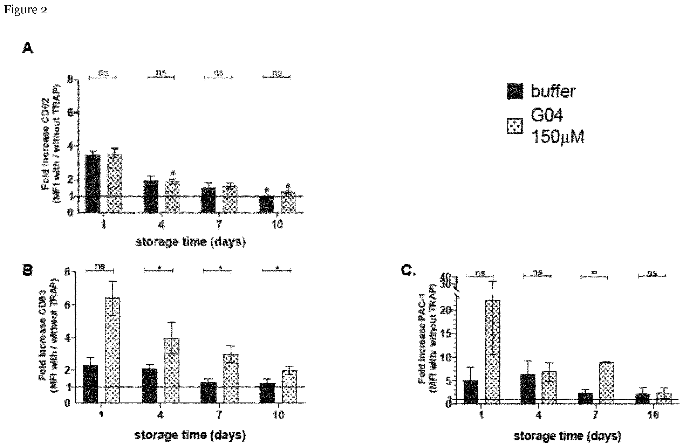

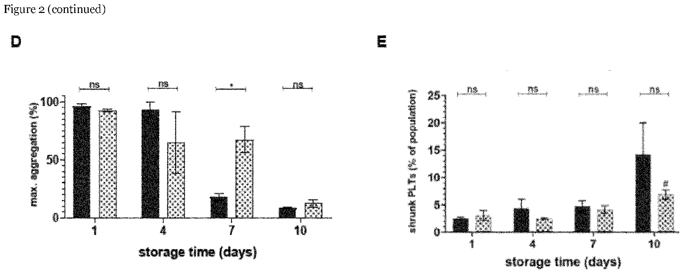

After significant improvement of PLT survival with addition of G04, the inventors analyzed further impact on PLT function. A significant increase in fold CD63 expression (Figure 2B) was observed at day 4 (MFI CD63 6.38 vs 2.32, p=0.0215), day 7 (MFI CD63 2.98 vs 1.25, p=0.035) and day 10 (MFI CD63 3.97 vs 1.24, p=0.049) after PLTs were treated with G04. Analysis of PAC-1 (Figure 2C) showed a significant increase at day 7 (MFI PAC1 8.82 vs 2.45, p=0.0047) but not on day 4 and day 10. Similarly, enhanced PLT aggregation (Figure 2D) was observed at day 7 (% max aggregation 67.33 vs 21.98, p=0.0186) in G04 treated PLTs. No significant differences were observed in the levels of CD62 expression and PLT shrinkage.

### Effect of PKA inhibition using forskolin on cold stored APCs

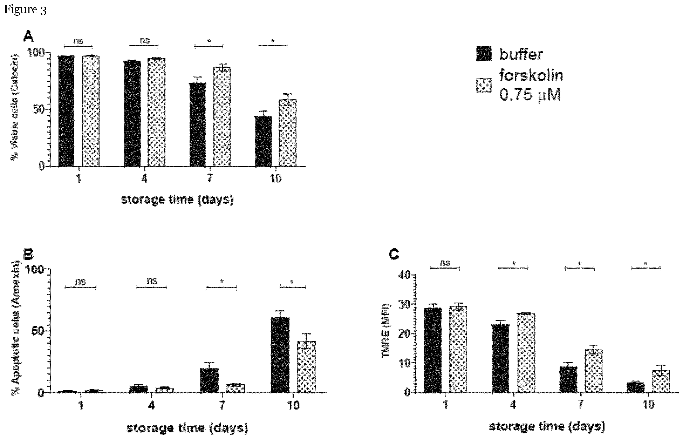

Furthermore, the inventors tested efficacy of forskolin, a clinically approved PKA inhibitor on platelet viability and apoptosis in cold stored APCs. APCs were incubated with and/or without 0.75 µM forskolin at 4°C with gentle agitation. A significant increase in viability was observed (Figure 3A) in forskolin treated PLTs at day 7 (% viable cells 87.1 vs 73.4 p=0.0416) and at day 10 (58.95 vs 44.46, p=0.044). In corroboration with this trend, forskolin treatment also significantly diminished PLT apoptosis at day 7 (% apoptotic cells 9.3 vs 17.6, p=0.0400) and even more strongly at day 10 (% apoptotic cells 33 vs 60.1, p=0.0020) (Figure 3B). Similar trends were observed in the measurement of mitochondrial transmembrane potential at day 7 and day 10 (ΔΨm MFI 17.88 vs 10.34, p=0.0131 and 11.02 vs 3.95, p=0.034 respectively) (Figure 3C).

### Effect of PKA inhibition on the function of cold storage APCs

Cold stored APCs incubated with forskolin were also analyzed for their functional parameters. No significant impact was found in any parameters utilized in our experiments. Although there was a tendency of increased PAC-1 expression after treatment with forskolin, it was not significant. A slightly enhanced aggregation was observed at day 4 but also in untreated APCs.

### Effect of Caspase-9 Inhibition on cold stored APCs:

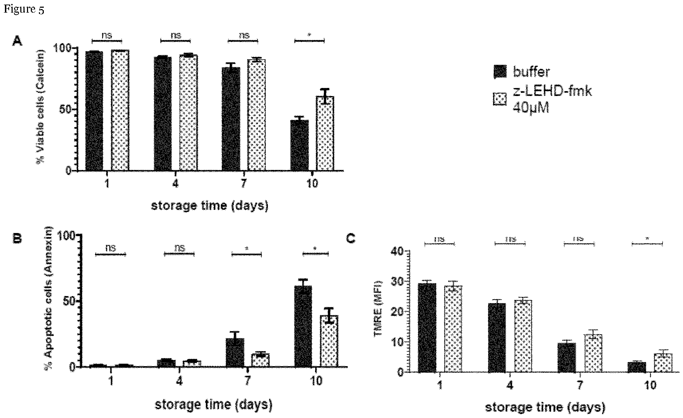

Along with G04 and forskolin, the inventors also included z-LEHD-fmk, a known inhibitor of Caspase-9 pathway with a demonstrated role in platelet apoptosis. z-LEHD-fmk also improved PLT viability and function of cold stored PLTs. An increased PLT viability (Fig 5A) was observed at day 10 (% viable cells 60.38 vs 41.04, p=0.0336) after treatment with z-LEHD-fmk. Analysis of apoptosis (Fig 5B) revealed decreased apoptotic cells at day 7 (% apoptotic cells 9.85 vs 21.69, p=0.038) and day 10 (% apoptotic cells 39.00 vs 61.12, p=0.0136) after the treatment. Mitochondrial transmembrane potential measurement (Fig 5C) showed an increase at day 10 (ΔΨm MFI 6.26 vs 3.34, p=0.0472).

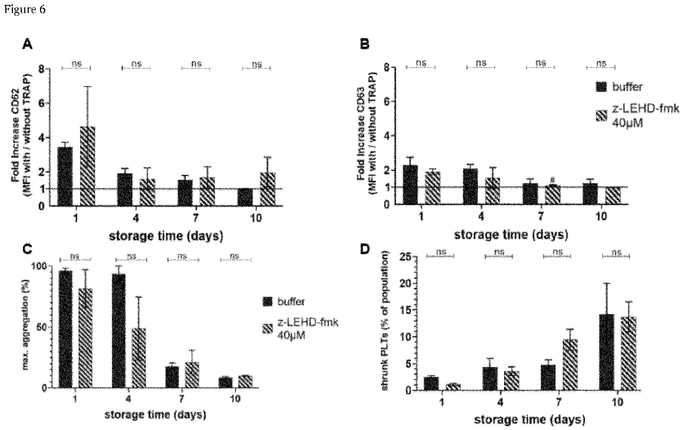

Functional analysis of cold stored APCs incubated with z-LEHD-fmk revealed no significant impact on any PLT parameters included in our invention. A tendency of decreased PLT aggregation was observed at day 4 after the treatment, but it was not significant (Figure 6C).

A summary of the results is shown in table 1 and 2.

**Table 1: Summary of the results, viability and apoptosis of 4°C stored APC**

| Compound | % PLT viability (day 10) | % Apoptosis (day 10) |
|---|---|---|
| RhoA inhibitor G04 | 60.82 | 33 |
| Phosphokinase A (PKA) activator forskolin | 58.95 | 33 |
| Caspase-9 inhibitor z-LEHD-fmk | 60.38 | 39 |

**Table 2: Summary of the results, PLT aggregation of 4°C stored APC**

| Compound | % PLT aggregation (day 7) |
|---|---|
| RhoA inhibitor G04 | 67.33 (increase) |
| Phosphokinase A (PKA) activator forskolin | No significant effect |
| Caspase-9 inhibitor z-LEHD-fmk | No significant effect |

### References as cited

1. Rosenfeld BA, Herfel B, Faraday N, Fuller A, Braine H: Effects of storage time on quantitative and qualitative platelet function after transfusion. Anesthesiology 1995;83: 1167-1172.
2. Brecher ME, Blajchman MA, Yomtovian R, Ness P, AuBuchon JP: Addressing the risk of bacterial contamination of platelets within the United States: a history to help illuminate the future. Transfusion 2013;53:221-231.
3. Dumont LJ, Gulliksson H, van der Meer PF, et al.: Interruption of agitation of platelet concentrates: a multicenter in vitro study by the BEST Collaborative on the effects of shipping platelets. Transfusion 2007;47:1666-1673.
4. Seghatchian J, Krailadsiri P: The platelet storage lesion. Transfus Med Rev 1997;11:130-144.
5. Capocelli KE, Dumont LJ: Novel platelet storage conditions: additive solutions, gas, and cold. Curr Opin Hematol 2014;21:491-496.
6. Devine DV, Serrano K: The platelet storage lesion. Clin Lab Med 2010;30:475-487.
7. Currie LM, Harper JR, Allan H, Connor J: Inhibition of cytokine accumulation and bacterial growth during storage of platelet concentrates at 4 degrees C with retention of in vitro functional activity. Transfusion 1997;37:18-24.
8. Sandgren P, Hansson M, Gulliksson H, Shanwell A: Storage of buffy-coat-derived platelets in additive solutions at 4 degrees C and 22 degrees C: flow cytometry analysis of platelet glycoprotein expression. Vox Sang 2007;93:27-36.
9. Reddoch KM, Pidcoke HF, Montgomery RK, et al.: Hemostatic function of apheresis platelets stored at 4 degrees C and 22 degrees C. Shock 2014;41 Suppl 1:54-61.
10. Pidcoke HF, Spinella PC, Ramasubramanian AK, et al.: Refrigerated platelets for the treatment of acute bleeding: a review of the literature and reexamination of current standards. Shock 2014;41 Suppl 1:51-53.
11. Montgomery RK, Reddoch KM, Evani SJ, Cap AP, Ramasubramanian AK Enhanced shear-induced platelet aggregation due to low-temperature storage. Transfusion 2013;53:1520-1530.
12. Lee DH, Blajchman MA: Novel treatment modalities: new platelet preparations and substitutes. Br J Haematol 2001;114:496-505.
13. Hoffmeister KM, Felbinger TW, Falet H, et al.: The clearance mechanism of chilled blood platelets. Cell 2003;112:87-97.
14. Murphy S, Gardner FH: Effect of storage temperature on maintenance of platelet viability--deleterious effect of refrigerated storage. N Engl J Med 1969;280:1094-1098.
15. Pidcoke HF, McFaul SJ, Ramasubramanian AK, et al.: Primary hemostatic capacity of whole blood: a comprehensive analysis of pathogen reduction and refrigeration effects over time. Transfusion 2013;53 Suppl 1:137S-149S.
16. Jobes D, Wolfe Y, O'Neill D, et al.: Toward a definition of "fresh" whole blood: an in vitro characterization of coagulation properties in refrigerated whole blood for transfusion. Transfusion 2011;51:43-51.
17. Valeri CR: Circulation and hemostatic effectiveness of platelets stored at 4 C or 22 C: studies in aspirin-treated normal volunteers. Transfusion 1976;16:20-23.
18. Sweeney J, Kouttab N, Holme S, Kurtis J, Cheves T, Nelson E: Storage of platelet-rich plasma-derived platelet concentrate pools in plasma and additive solution. Transfusion 2006;46:835-840.
19. Slichter SJ, Corson J, Jones MK, et al.: Exploratory studies of extended storage of apheresis platelets in a platelet additive solution (PAS). Blood 2014;123:271-280.
20. Hornsey VS, McColl K, Drummond O, et al.: Extended storage of platelets in SSP platelet additive solution. Vox Sang 2006;91:41-46.
21. Thiele T, Pohler P, Kohlmann T, et al.: Tolerance of platelet concentrates treated with UVC-light only for pathogen reduction--a phase I clinical trial. Vox Sang 2015;109:44-51.
22. Bakchoul T, Fuhrmann J, Chong BH, Bougie D, Aster R, Subcommittee on Platelet I: Recommendations for the use of the non-obese diabetic/severe combined immunodeficiency mouse model in autoimmune and drug-induced thrombocytopenia: communication from the SSC of the ISTH. J Thromb Haemost 2015;13:872-875.
23. Fuhrmann J, Jouni R, Alex J, et al.: Assessment of human platelet survival in the NOD/SCID mouse model: technical considerations. Transfusion 2016;56:1370-1376.
24. Jansen AJ, Josefsson EC, Rumjantseva V, et al.: Desialylation accelerates platelet clearance after refrigeration and initiates GPIbalpha metalloproteinase-mediated cleavage in mice. Blood 2012;119:1263-1273.
25. Wandall HH, Hoffmeister KM, Sorensen AL, et al.: Galactosylation does not prevent the rapid clearance of long-term, 4 degrees C-stored platelets. Blood 2008;111:3249-3256.
26. van der Wal DE, Du VX, Lo KS, Rasmussen JT, Verhoef S, Akkerman JW: Platelet apoptosis by cold-induced glycoprotein Ibalpha clustering. J Thromb Haemost 2010;8:2554-2562.
27. van der Wal DE, Verhoef S, Schutgens RE, Peters M, Wu Y, Akkerman JW: Role of glycoprotein Ibalpha mobility in platelet function. Thromb Haemost 2010;103:1033-1043.
28. Chen W, Druzak SA, Wang Y, et al.: Refrigeration-Induced Binding of von Willebrand Factor Facilitates Fast Clearance of Refrigerated Platelets. Arterioscler Thromb Vasc Biol 2017;37:2271-2279.
29. Shiri R, Yari F, Ahmadinejad M, Vaeli S, Tabatabaei MR: The caspase-3 inhibitor (peptide Z-DEVD-FMK) affects the survival and function of platelets in platelet concentrate during storage. Blood Res 2014;49:49-53.
30. Slichter SJ, Bolgiano D, Corson J, et al.: Extended storage of buffy coat platelet concentrates in plasma or a platelet additive solution. Transfusion 2014;54:2283-2291.

## Claims

1. A pharmaceutical composition for cold storing blood platelets, comprising platelets in a suitable storage medium, and at least one inhibitor of platelet apoptosis.

2. The pharmaceutical composition according to claim 1, wherein said cold storing is at a temperature of between about 0°C to about 10°C, preferably between about 2°C to about 5°C, and more preferably at about 4°C.

3. The pharmaceutical composition according to claim 1 or 2, wherein said blood platelets are from a mammalian donor, in particular a human donor.

4. The pharmaceutical composition according to any one of claims 1 to 3, comprising platelets as a blood platelet concentrate, such as an autologous or apharesis platelet concentrate (APC), platelets in additive solutions (PAS) or a platelet-rich plasma (PRP), wherein preferably said PRP does not comprise more than about 35% of plasma.

5. The pharmaceutical composition according to any one of claims 1 to 4, wherein said apoptosis inhibitor is a selective inhibitor targeting cold-storage induced apoptosis, wherein preferably said apoptosis inhibitor is selected from the group of specific RhoA subfamily Rho GTPases inhibitors, RhoA inhibitor G04, specific phosphokinase A (PKA) activators, forskolin, specific caspase-9 inhibitors, z-LEHD-fmk, and combinations thereof, in particular RhoA inhibitor G04 combined with forskolin.

6. The pharmaceutical composition according to any one of claims 1 to 5, wherein said cold stored blood platelets show a % platelet (PLT) viability on day 10 of more than about 50%, preferably more than about 55%, more preferably more than about 60%.

7. The pharmaceutical composition according to any one of claims 1 to 6, wherein said cold stored blood platelets show a % apoptosis on day 10 of less than about 40%, and preferably less than about 35%.

8. The pharmaceutical composition according to any one of claims 1 to 7, wherein said cold stored blood platelets show a % PLT aggregation on day 7 of more than about 60%, and preferably more than about 65%.

9. A method for preparing a pharmaceutical composition according to any one of claims 1 to 8, comprising providing a sample of blood platelets to be cold-stored in a suitable storage medium, and adding to said blood platelets at least one inhibitor of platelet apoptosis.

10. The method according to claim 9, wherein said blood platelets are from a mammalian donor, in particular a human donor.

11. The method according to claim 9 or 10, wherein said blood platelets are a blood platelet concentrate, such as an autologous or apharesis platelet concentrate (APC), platelets in additive solutions (PAS) or a platelet-rich plasma (PRP), or derived therefrom, and wherein preferably said PRP does not comprise more than about 35% of plasma.

12. The method according to any one of claims 9 to 11, wherein said apoptosis inhibitor is a selective inhibitor targeting cold-storage induced apoptosis, wherein preferably said apoptosis inhibitor is selected from the group of specific RhoA subfamily Rho GTPases inhibitors, RhoA inhibitor G04, specific phosphokinase A (PKA) activators, forskolin, specific caspase-9 inhibitors, z-LEHD-fmk, and combinations thereof, in particular RhoA inhibitor G04 combined with forskolin.

13. The method according to any one of claims 9 to 12, further comprising the step of cold-storing said pharmaceutical composition, wherein said cold storing is at a temperature of between about 0°C to about 10°C, preferably between about 2°C to about 5°C, and more preferably at about 4°C.

14. The method according to claim 13, wherein said cold-storing of the pharmaceutical composition is for more than about 4 days, preferably for more than about 7 days, more preferably for more than about 10 days, and most preferably for more than about 14 days.

15. The pharmaceutical composition according to any one of claims 1 to 8 for use in the prevention and/or treatment of diseases, preferably for use in platelet transfusion.
